# EUROPEAN PATENT APPLICATION

(11) **EP 4 576 116 A1**
(43) Date of publication of application: **25.06.2025**
(21) Application number: 23220087.3
(22) Date of filing: 22.12.2023
(51) Int. Cl.: G16H 40/63, A61B 5/00, G06N 3/045, G16H 50/20

(54) **SYSTEM FOR PLANNING AND/OR PROVIDING NEUROMODULATION AND/OR NEUROSTIMULATION AND METHOD THEREOF**

(71) Applicant: Ecole Polytechnique Fédérale de Lausanne (EPFL), 1015 Lausanne (CH)
(72) Inventor: Sakr, Icare, 1007 Lausanne (CH); Lorach, Henri, 1094 Paudex (FR); Courtine, Grègoire, 1003 Lausanne (CH); Alahi, Alexandre, 1095 Lutry (CH)
(74) Representative: DTS Patent- und Rechtsanwälte PartmbB

(57) **Abstract**

The present invention provides a system (100) for planning and/or providing control to neuromodulation and/or neurostimulation and/or an actuator such as a brain computer interface (BCI) and/or providing a neural interface system, especially a brain-spinal-cord-interface system, the system (100) comprising: at least one input module (10) for brain signals (S), especially raw brain signals (S); at least one pre-processing module (12) for converting the raw brain signals (S) into input tensors, wherein the input tensors include at least one of temporal and/or spatial and/or spectral information, and at least one conversion module (13) comprising an encoder and decoder network architecture (14), wherein the encoder and decoder network architecture (14) is configured to be trained by using partially masked samples of the input tensors to reconstruct electrical activity of a given representation of the input tensors, wherein a masking ratio is applied with positional embedding, which provides simultaneous learning of local and global features present in the brain data, thereby transforming the input tensors into a latent space. The invention further provides a method for planning neuromodulation and/or neurostimulation through the above-described system (100).

## Description

The present invention belongs to the technical field of neuromodulation and/or neurostimulation, e.g. for restoring and/or controlling a motor function in in a subject in need thereof.

In particular, said subject can be a human patient affected by spinal cord injury (SCI) and/or other neurological disorders such disorders such as stroke, Parkinson's disease, and/or other neurodegenerative disorders.

In particular, the present invention provides an improved system for planning and/or providing a neuromodulation and/or neurostimulation.

The invention further provides a method for planning neuromodulation and/or neurostimulation through said system.

Still further, the invention provides a computer-readable non-volatile storage medium comprising computer-readable instructions that, when executed by a processing means, causes said processing means to implement said method.

Neuromodulation and/or neurostimulation can be provided for restoring and/or controlling motor functions of a patient, e.g. movements of the upper and/or lower limbs.

Here, it is important that delivered stimulation is well-synchronized with a movement intention of the patient, thereby providing a feeling that is close to natural as much as possible.

The use of brain-computer interfaces (BCI) is known to help for this purpose.

In particular, BCI rely on extraction and interpretation of brain activity to control a wide variety of effectors.

However, despite the growing amount of collected neurophysiological data, classical machine learning methods still fail to provide robust and transferable models to effectively support clinically relevant applications.

Decoding movement intentions involves extracting useful information from raw sequences of neural activity and linking it to specific behaviors, thus establishing a conversion route from thoughts to actions.

Decoders are typically trained using supervised machine learning algorithms.

However, numerous challenges arise in this process, notably characterized by distribution shifts in neural dynamics across times, tasks, subjects, and therapeutic modalities.

The fundamental problem lies in the difficulty of annotating brain activity compared to other domains of artificial intelligence.

Typically, data labeling is performed through calibration sessions involving repetition of numerous instructed intentions within a controlled clinical environment.

Apart from being expensive to obtain, labels are often uncertain and compressed descriptions of neural activity.

Consequently, the trained decoders rely on task-specific characteristics, such that even minor deviations from the task conditions may significantly impair the performance, necessitating constant recalibration.

Conversely, unlabeled data can be readily obtained for extended periods in everyday life when the participant is not following explicit instructions.

This renders this option potentially interesting to explore when restoring and/or controlling of motor functions of a patient are concerned.

Starting from this notion, the inventors investigated the possibility of effectively extracting meaningful information from the abundant unlabeled cortical data, and acquiring generic brain features with transferable properties that require minimal calibration for extension to differing contexts.

Self-supervised learning (SSL) has recently emerged as a powerful approach for learning intelligible representations of data without the need for explicit labels.

Unlike supervised learning, which largely depends on carefully annotated data and is limited by its availability, self-supervised approaches have the advantage of being able to leverage vast quantities of unlabeled data to learn generalist models that can be adapted to various tasks.

SSL has played a pivotal role in significant achievements of artificial intelligence in Natural Language Processing (NLP), ranging from machine translation to the development of large language models trained on vast corpora **[1]-[4].**

SSL has also pushed new boundaries in computer vision, where SSL-pre-trained models were able to outperform models trained on labels **[5].**

In particular, the main concept behind SSL is to learn informative representations by defining a pretext task that derives the supervision signal from the input itself.

In the field of NLP, a frequently used SSL task involves a reconstructive objective, e.g. hiding words within a sentence and training a neural network to predict the missing words based on the surrounding context.

Through such unsupervised interactions with the data, the model acquires a general-istic understanding of the fundamental structure and intricacies of language.

These learned representations can subsequently be transferred to a diverse range of downstream tasks, e.g. including text summarization, translation, and even human interaction, e.g. ChatGPT.

This masking objective was also used in the field of computer vision, termed "masked image modeling", by similarly masking portions of an image, and training a model to recover the original image **[6], [7].**

Also, this approach has been recently extended to learning self-supervised representations of audio spectrograms **[8].**

These masked autoencoders demonstrated unprecedented capacity to simultaneously learn local and global structures in the data and to achieve competitive performances on a variety of vision and speech tasks.

The opportunity of transferring these principles to BCI applications appears particularly beneficial, in particular because acquiring comprehensive labels is often challenging and/or specific tasks may not be known in advance.

Recent efforts have evaluated SSL strategies in neuro-physiological data such as EEG **[9]-[12],** neural population spikes **[13], [14],** or even across modalities **[15].**

For example, Schneider et al. **[15]** describes the use of a contrastive SSL objective to infer consistent neural embeddings by implicitly labeling over time.

Contrastive learning consists of creating a contrast in latent space by enforcing variance between incompatible or "negative" samples (e.g., samples distant in time) and promoting invariance between similar or "positive" samples (e.g., samples close in time).

In particular, these studies provide a preliminary evidence that an SSL-trained encoder could handle some neural variabilities and generate representations that outperform supervised learning in downstream tasks, in particular when there is a limited amount of labeled data available.

However, these known approaches still fail to demonstrate sufficient evidence of their ability to effectively generalize to novel tasks and/or conditions that were not included in the training set.

Additionally, these known approaches have proven uncapable of leveraging the potential of SSL to overcome distribution shifts in clinically relevant settings.

A partial solution to the aforementioned drawbacks is provided in Wang et al. **[16],** teaching the use of a masked reconstructive objective on time-frequency representations (spectrograms) of intracranial electrodes.

This solution is adapted to learn unsupervised brain representations that improve decoding accuracy and sample efficiency across tasks involving patients listening to audio stimuli, and can generalize to different subjects.

Nonetheless, there is still room for further improvement.

In the light of the above, it is an object for the present invention to provide a system for planning and/or providing neuromodulation and/or neurostimulation, capable of harvesting unlabeled brain data to learn robust features from electrical activity across time, tasks, patients and/or recording modalities, thereby allowing providing stimulation in a more effective and targeted way.

The above-specified object is achieved by the provision of a system as defined in claim 1.

According to the invention, a system for planning and/or providing control to neuromodulation and/or neurostimulation and/or an actuator such as a brain computer interface (BCI) and/or providing a neural interface system, especially a brain-spinal-cord-interface system, is provided, said system comprising:
- at least one input module for brain signals, especially raw brain signals,
- at least one pre-processing module for converting the raw brain signals into input tensors, wherein the input tensors include at least one of temporal and/or spatial and/or spectral information, and
- at least one conversion module comprising an encoder and decoder network architecture,
- wherein the encoder and decoder network architecture is configured to be trained by using partially masked samples of the input tensors to reconstruct electrical activity of a given representation of the input tensors, wherein a masking ratio is applied with positional embedding, which provides simultaneous learning of local and global features present in the brain data, thereby transforming the input tensors into a latent space.

Also provided is a method for planning neuromodulation and/or neurostimulation through the above-described system.

The present invention provides a system for planning and/or providing neuromodulation and/or neurostimulation for a subject in need thereof.

Preferably, said subject is a human patient, e.g. affected by spinal cord injury (SCI) and/or other neurological disorders such as stroke, Parkinson's disease, and/or other neurodegenerative disorders.

The system comprises at least one input module for brain signals.

For instance, the system may comprise a single input module.

Alternatively, the system may comprise a plurality of input modules, according to the needs.

Especially, said brain signals are raw brain signals.

Preferably said brain signals are electrophysiological raw brain signals.

The at least one input module can be configured and adapted for recording brain signals through invasive techniques.

Alternatively, the at least one input module can be configured and adapted for recording brain signals through non-invasive techniques.

The system further comprises at least one pre-processing module. The at least one pre-processing module is adapted for converting the raw brain signals into input tensors.

For instance, the system may comprise a single pre-processing module.

Alternatively, the system may comprise a plurality of pre-processing modules, according to the needs.

In particular, the input tensors may include temporal information.

Additionally or alternatively, the input tensors may include spatial information.

Additionally or alternatively, the input tensors may include spectral information.

Preferably, a 3D masking strategy is implemented, randomly masking patches of spatial, spectral and temporal information.

This allows addressing the drawbacks of known approaches relying on 2D masking, which are restricted to entire frequency or time bands.

Additionally, 3D positional embeddings of the input can be incorporated, making the neural network aware of the spatial positions of electrodes relative to the brain atlas, for each patient.

This approach is beneficial in particular for the purpose of transferring knowledge across patients.

The system further includes at least one conversion module. The at least one conversion module comprises an encoder and decoder network architecture.

For instance, the system may comprise a single conversion module.

Alternatively, the system may comprise a plurality of conversion modules, according to the needs.

In particular, the encoder and decoder network architecture is configured to be trained by using partially masked samples of the input tensors to reconstruct a given representation of the input tensors.

In particular, a masking ratio is applied with positional embedding, which provides simultaneous learning of local and global features present in the brain data, thereby transforming the input tensors into a latent space.

The invention is based on the basic idea that, by transforming the input tensors into a latent space, it is possible provide the most relevant and robust features to predict behaviors including motor intentions, sensory or physiological state. In particular, the system of the invention is able to learn this transformation to produce generic features that can handle clinically relevant decoding challenges by accumulating background knowledge about the brain in an unsupervised manner.

Accordingly, decoding predictions can be improved with smaller number of labelled data and provide reliable inputs to a neuroprosthetic system to restore function.

Advantageously, latent features that are present in the brain data can be used in downstream tasks such as motor intention classification, compensation of temporal drift or patient-to-patient generalization.

Brain signals can be captured either through experimental sessions or in everyday life conditions.

The system according to the invention is able to efficiently model the dynamics of change of brain signals over time.

This knowledge can be used to enhance robustness of decoding against long-term shifts in neural dynamics.

Advantageously, the encoder and decoder network architecture may be configured to perform self-supervised learning (SSL).

The use of SSL concepts in this context is beneficial since acquiring comprehensive labels is often challenging, and/or one or more specific tasks may not be known in advance.

Advantageously, the encoder and decoder network architecture is configured to learn patterns of unlabeled neural activity.

In particular, by learning patterns in unlabeled neural activity, it is possible to at least partially uncover the underlying dynamical structure of the brain, and establish a generic latent space that correlates with behavior in varying contexts.

Otherwise stated, by learning the dynamical structure of the brain, it is possible to reliably predict behavior.

Also, this approach allows overcoming difficulties and drawbacks underlying known solutions broadly relying on labeled data in annotating activities of the brain.

As mentioned, labels are often uncertain and compressed descriptions of neural activity, so that trained decoders are only enabled to rely on task-specific characteristics.

Accordingly, even minor deviations from the specific task conditions may significantly impair the performance, requiring constant recalibration.

To the contrary, unlabeled data can be readily obtained for extended periods, even in situations of everyday life while the patient is not performing a specific task and/or following explicit instructions.

Advantageously, said brain signals may at least partially comprise cortical brain signals.

Preferably, said signals at least partially comprise unlabeled cortical brain signals.

Advantageously, the input tensors are 3D input tensors.

Preferably, said 3D input tensors have a spatial and/or temporal and/or spectral dimension, thereby preserving the temporal, spatial and spectral coherence of information.

Incorporating information in these 3 dimensions of logic guides the neural network to learn patterns specific to each logic, thereby increasing its efficiency and interpretability.

Advantageously, the pre-processing module is configured to use a wavelet transformation to convert the brain signals into input tensors.

The use of a wavelet transformation is particularly advantageous for the implementation of real time computation and preservation of temporal information.

Advantageously, the encoder and decoder network architecture may comprise an encoder module.

In particular, said encoder module is configured to transform the brain signals into a latent space.

There may be also a decoder module provided in the encoder and decoder network architecture.

In particular, said decoder module is configured to map the latent space to the original feature space.

The decoder module is also configured to compute the reconstruction loss.

Thus, after SSL pretraining, the learned latents can be used to visualize brain trajectories, estimate domain shifts and provide robust input features to predict behavior.

In particular, self-supervised pretraining utilizes long-term data including resting states and a range of behavioral tasks, thereby overcoming the limitations of known approaches only relying on single-task data.

By avoiding relying on task-specific data, it is possible to enhance robustness and generalizability of a model to a variety of new tasks that have not been observed during training.

Advantageously, said masking ratio is a high masking ratio with positional embedding.

A high masking ratio with positional embedding enables the simultaneous learning of local and global features present in the brain data.

In particular, the provision of a high masking ratio in the self-supervised pretraining task largely reduces redundancy, encouraging the neural network to learn informative solutions that effectively capture high-level brain features.

Conversely, a low masking ratio - given the highly correlated nature of multi-channeled brain signals - only allows for the learning of trivial solutions, that interpolate the masked patches from neighboring contexts in the input tensors, without learning interesting representations.

Further, positional embeddings enable the neural network to use certain position information (such as spatial, spectral and/or temporal positions) of the input tensor as a guiding signal for the simultaneous learning of local and global features.

The system according to the invention can be used in online, real-time clinical settings.

The present invention further provides a method for planning neuromodulation and/or neurostimulation through the system described above.

In particular, the method comprises:
- providing at least one input module for brain signals, especially raw brain signals, preferably wherein said brain signals at least partially include cortical brain signals, more preferably unlabeled cortical brain signals,
- converting the raw brain signals into input tensors, preferably 3D input tensors, through a pre-processing module, said input tensors including at least one of temporal and/or spatial and/or spectral information,
- providing at least one conversion module, said conversion module comprising an encoder and decoder network architecture,
- training the encoder and decoder network architecture by using partially masked samples of the input tensors to reconstruct a given representation of the input tensors.

A masking ratio is applied with positional embedding, which provides simultaneous learning of local and global features present in the brain data, thereby transforming the input tensors into a latent space.

This enables visualizing brain trajectories and estimating domain shifts to provide the most relevant and robust input features to predict behavior.

Advantageously, the method may include implementing a 3D masking strategy, randomly masking patches of spatial, spectral and temporal information.

Additionally, the method may include incorporating 3D positional embeddings of the input, making the neural network aware of the spatial positions of the electrodes relative to the brain atlas, for each patient.

Preferably, said brain signals at least partially include cortical brain signals.

More preferably, said brain signals at least partially include unlabeled cortical brain signals.

Preferably, said input tensors are 3D input tensors.

Preferably, the dimensions of the 3D input tensors include at least one of spatial, temporal and/or spectral information.

Advantageously, the method may comprise performing a self-supervised learning (SSL).

This step is implemented through the encoder and decoder network architecture described above.

Advantageously, the method may comprise:
- learning patterns of unlabeled neural activity, thereby at least partially uncovering the underlying dynamical structure of the brain, and
- establishing a generic latent space that correlates with behavior in varying contexts.

These steps are also implemented through the encoder and decoder network architecture described above.

Advantageously, the step of converting the brain signals into input tensors may be implemented using a wavelet transformation.

Advantageously, the method may comprise transforming the brain signals into a latent space.

This step is implemented through the encoder module described above.

Here, the method may also comprise:
- mapping the latent space to the original feature space, and
- computing the reconstruction loss.

These steps are implemented through the decoder module described above.

Finally, the invention provides a computer-readable non-volatile storage medium comprising computer-readable instructions that, when executed by a processing means, causes said processing means to implement the method steps described above.

Further details and advantages of the present invention shall now be disclosed in connection with the drawings, where:

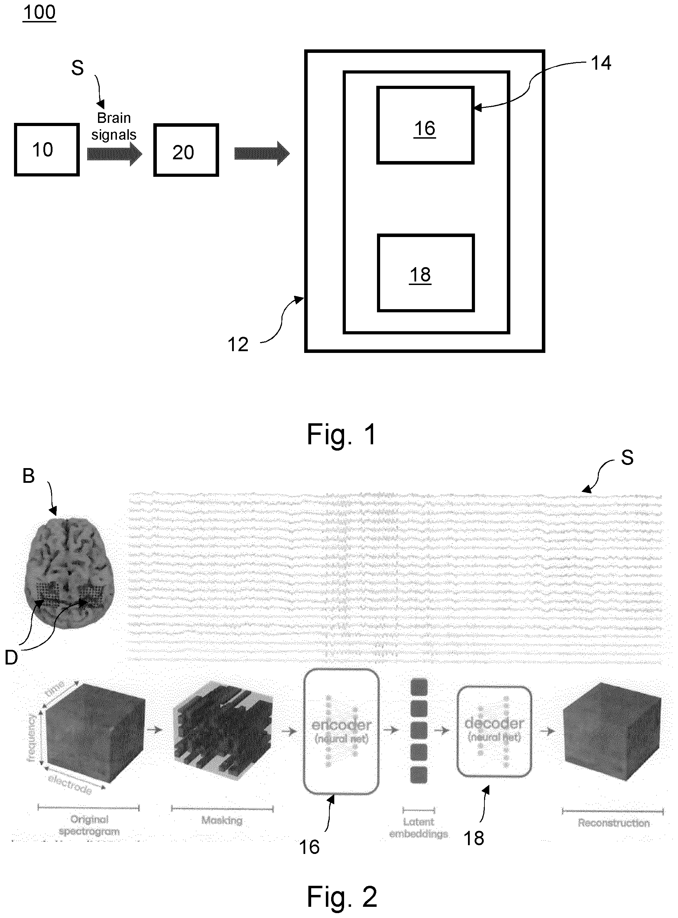

**Fig. 1** is a block diagram schematically showing a system for planning and/or providing neuromodulation and/or neurostimulation to a subject, e.g. a human patient in need thereof, according to an embodiment of the invention.
**Fig. 2** is a diagram showing how an encoder and decoder network architecture of the system of **Fig. 1** implements learning of self-supervised brain representations. Here, brain cortical recordings capture the electrical activity of the neurons. This activity reflects the behavior and intentions of the patient. It can be decomposed in spatial, spectral and temporal features. The system learns the internal structure of this activity by trying to reconstruct the full data from a fraction of it. The later embedding can be viewed as a compressed version of the input that is more informative and provides higher performance in downstream tasks such as movement classification and regression.

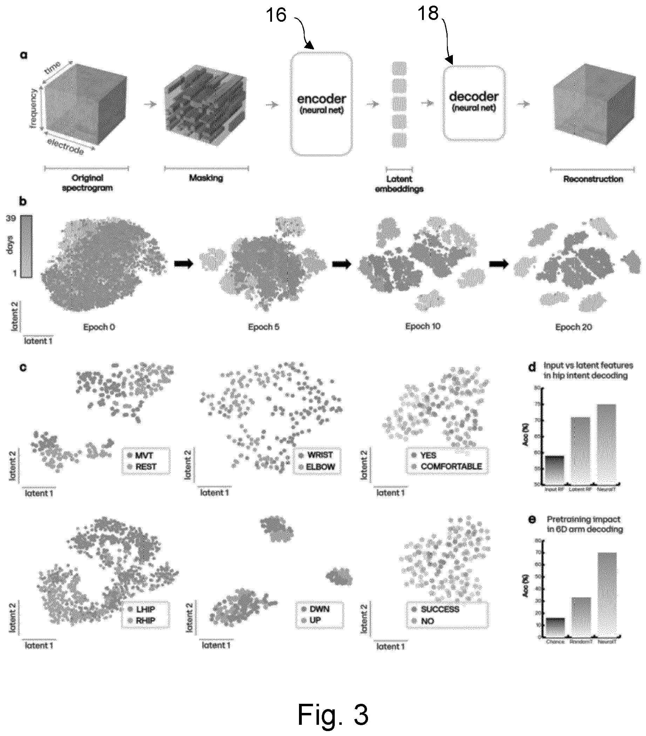

**Fig. 3** is a diagram showing how informative natural embeddings are uncovered in the system of the invention. In particular: **a.** masked auto-encoder framework to learn self-supervised brain representations. Transformer encoder-decoder neural networks learn to restore the spatio-spectro-temporal features from their sparse sampling; **b.** t-distributed stochastic neighbor embedding t-SNE projection of the learned latents with pre-training epochs across time, with each data point colored according to the day after implantation; **c.** t-SNE projection of the learned latents across behavioral tasks, unobserved during training, showing a modeling of lower limb, upper limb, and speech brain concepts; **d.** performance in decoding hip movement intentions (33.3% chance) on a moment-by-moment basis with data points of 200 ms in length. A Random Forest (RF) decoder trained on the latent features (Latent RF), gains 12 points compared to when trained on the input wavelet features (InputRF), with only 4% difference to a transformer decoder (NeuralT), suggesting that meaningful feature extraction is key; **e.** performance in 6-class arm direction decoding (16.7% chance) with limited labels (10 per class), of a randomly initialized transformer (RandomT) and an SSL-pre-trained transformer (NeuraIT).

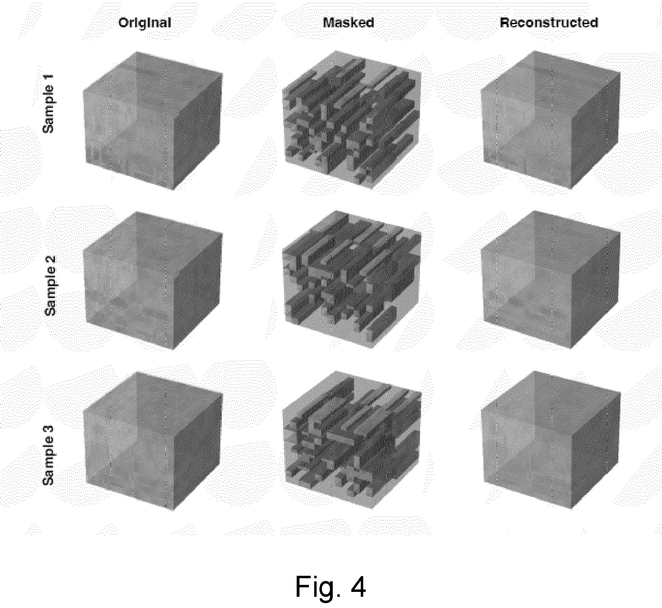

**Fig. 4** is a diagram schematically illustrating the network's reconstruction of three brain activity samples, including spatio-spectro-temporal information, from their randomly masked sampling, through the system of the invention.

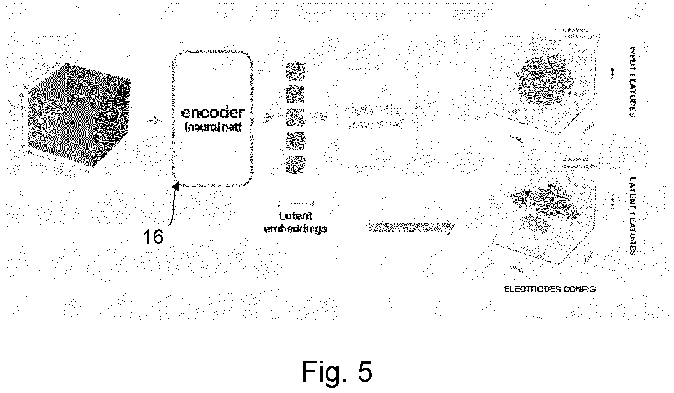

**Fig. 5** is a diagram schematically illustrating the projection of the latent and the input features in 3D using t-SNE, which is a non-linear dimensionality reduction method. The latent features reveal meaningful structures (here, different electrode configurations), that are less visible in the input features.

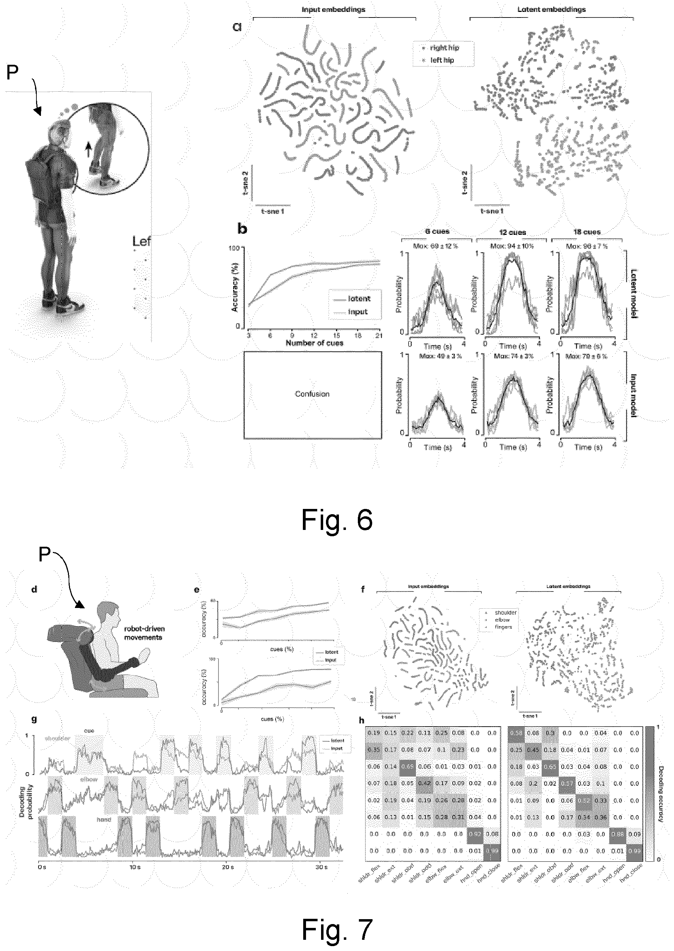

**Fig. 6** is a diagram showing how the system of the invention allows achieving highly-decodable and efficient feature extraction. Here, the example refers to a task where a patient was attempting movements of the lower limbs, in particular walking; In particular: **a.** t-SNE projection of the original input features and the latent features showing increased segregation in the latent embeddings of the left and right hip movement intentions; **b.** On the left, performance in hip movement intention decoding (33.3% chance) with increasing number of training cues. A classical decoder (Random Forest), trained on the latent features, shows increased performance compared to when trained on the input features, especially with low number of training labels. On the right, decoding probability peaks after movement onsets, showing that the decoder trained on the latent features, provides more confident predictions compared to the input model, even when the number of given cues is low.
**Fig. 7** is a diagram also showing how the system of the invention allows achieving highly-decodable and efficient feature extraction. Here, the example refers to an upper-limb motor decoding task with a spinal-cord injured patient. In particular: **d.** experimental setup of the task involving a robotic arm assisting the patient's movements; **e.** Performance of a Random Forest (RF) decoder trained on the latent features or the input features as a function of the percentage of the available training cues; **f.** t-SNE projection of the original input features and the latent features showing increased segregation in the latent embeddings corresponding to different upper limb joints involved in the movement; **g.** Decoding probabilities across time in three different states (shoulder, elbow, hand); **h.** Confusion matrices in 8 states upper limb classification of a RF trained on either the latent or the input features, showing a gain of 13 points in accuracy when using the latent features.

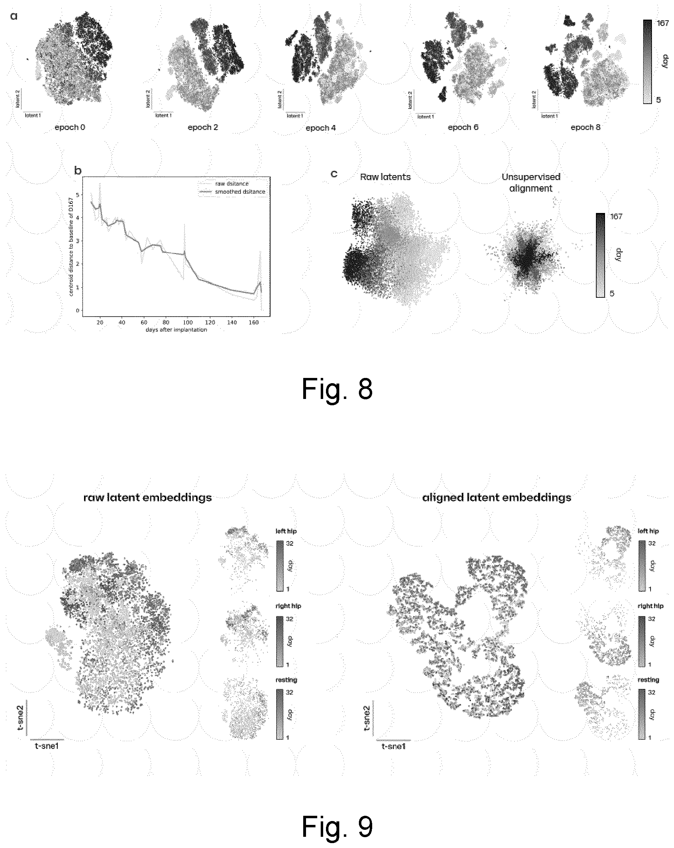

**Fig. 8** is a diagram showing how the latent features effectively model distribution shifts across time . In particular: **a.** t-SNE projection of the learned latents with pre-training epochs across time, with each data point colored according to the day after implantation; **b.** distance in latent space, of each day's centroid, relative to the centroid of day 167 after implantation; **c.** PCA projection of the learned latents before and after unsupervised alignment of second order statistics, with each data point colored according to the day after implantation;
**Fig. 9** is a diagram showing the t-SNE projections of the learned latents before and after supervised alignment using a contrastive objective based on the behavioral labels of left hip, right hip, and resting state. The left portion of the fig-ure illustrates the non-aligned latent features that show a change in the neural dynamics across days for the different behavioral conditions. The right portion of the figure illustrates the latent space obtained after contrastive alignment trained on day 1 to day 10 of rehabilitation (projected from day 1 to day 32 of rehabilitation), suggesting the possibility of obtaining a latent space that is robust and invariant to shifts in neural distributions from session to session.

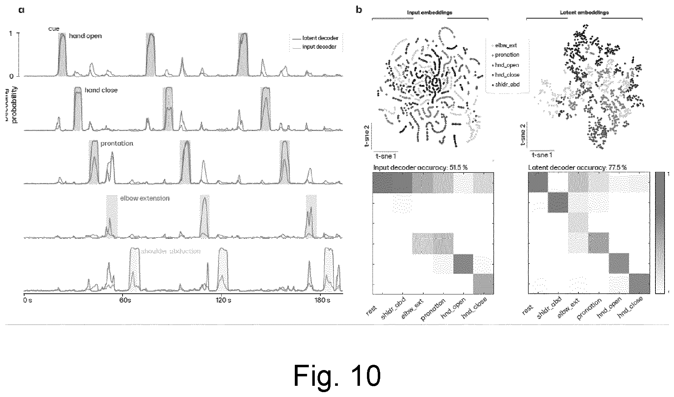

**Fig. 10** is a diagram showing experimental evidence on the ability of the system of the invention to learn a complex 6-states upper limb task based on latent features in online, real-time clinical settings.

**Fig. 1** provides a schematic overview of a system 100 for planning and/or providing control to neuromodulation and/or neurostimulation and/or an actuator such as a brain computer interface (BCI) and/or providing a neural interface system, especially a brain-spinal-cord-interface system according to an embodiment of the present invention.

Preferably, the system 100 may be used for restoring and/or controlling a motor function in a subject in need thereof.

Preferably, said subject is a human patient P affected by spinal cord injury (SCI) and/or other neurological disorders such as stroke, Parkinson's disease, and/or other neurodegenerative disorders.

The system 100 comprises at least one input module 10 for brain signals S, especially raw brain signals S, preferably be electrophysiological raw brain signals.

In the shown embodiment, the system 100 comprises a single input module 10 (**Fig**. **1**).

Not shown is that the system 100 may comprise a plurality of input modules 10, according to the needs.

The at least one input module 10 can be configured and adapted for recording brain signals through invasive techniques.

Alternatively, the at least one input module10 can be configured and adapted for recording brain signals through non-invasive techniques.

The system further comprises a pre-processing module 20.The system 100 further comprises at least one pre-processing module 20.

In the shown embodiment, the system 100 comprises a single pre-processing module 20 (**Fig. 1**).

Not shown is that the system 100 may comprise a plurality of pre-processing module 20s, according to the needs.

The pre-processing module 20 is configured to convert the raw brain signals S into input tensors.

In the present embodiment, said input tensors are 3D input tensors.

In particular, said input tensors include at least one of temporal and/or spatial and/or spectral information.

The system further comprises a conversion module 12.

In the shown embodiment, the system 100 comprises a single conversion module 12 (**Fig. 1**).

Not shown is that the system 100 may comprise a plurality of conversion modules 12, according to the needs.

The at least one conversion module 12 comprises an encoder and decoder network architecture 14 (**Fig. 1**).

In particular, the encoder and decoder network architecture 14 is configured to be trained by using partially masked samples of the input tensors, representing the brain's electrical activity, to reconstruct a given representation of the input tensors.

A masking ratio is applied with positional embedding, which provides simultaneous learning of local and global features present in the brain data, thereby transforming the input tensors into a latent space.

Preferably, a 3D masking strategy is implemented, randomly masking patches of spatial, spectral and temporal information, preferably at a high masking ratio.

A high masking ratio with positional embedding enables simultaneous learning of local and global information.

In particular, by masking tensors at a high ratio, it is ensured that high-level and interesting representations are learned, rather than trivial interpolation.

In the present embodiment, the encoder and decoder network architecture 14 is configured to perform self-supervised learning (SSL).

Also, in the present embodiment, the encoder and decoder network architecture 14 is configured to learn patterns of unlabeled neural activity.

This allows at least partially uncovering the underlying dynamical structure of the brain B, and establishing a generic latent space that correlates with behavior in varying contexts.

In the present embodiment, the brain signals S comprise at least partially cortical brain signals S, especially unlabeled cortical brain signals.

In the present embodiment, the pre-processing module 20 is advantageously configured to use a wavelet transformation to convert the brain signals S into input tensors.

In the present embodiment, the encoder and decoder network architecture 14 comprises an encoder module 16 (**Fig. 1**).

In particular, the encoder module 16 is configured to compress brain signals S into a latent space.

There is also a decoder module 18 provided in the encoder and decoder network architecture 14 (**Fig. 1**).

In particular, the decoder module 18 is configured to map the latent space to the original feature space and to compute the reconstruction loss.

By transforming the input tensors into a latent space, it is possible to visualize brain trajectories and estimate domain shifts to provide the most relevant and robust input features to predict behavior.

**Fig. 2** schematically illustrates how the encoder and decoder network architecture 14 implements learning of self-supervised brain representations.

Here, brain recording devices D, e.g. recording electrodes, preferably epidural recording electrodes, are used to capture electrical activity of the neurons.

This activity reflects the behavior and intentions of the patient P. It can be decomposed in spatial, spectral and/or temporal features.

The system 100 learns the internal structure of this activity by trying to reconstruct the full data from a fraction of it.

The latent embedding can be viewed as a compressed version of the input that is more informative and provides higher performance in downstream tasks, such as movement classification and regression.

**Fig. 3** schematically illustrates how uncovering informative neural embeddings is implemented in the system 100 according to the present embodiment.

In particular, **Fig. 3** shows the following:
**a.** masked auto-encoder framework to learn self-supervised brain represe tations. Transformer encoder-decoder neural networks learn to restore the spatio-spectro-temporal features from their sparse sampling;
**b.** t-SNE projection of the learned latents with pre-training epochs across, with each data point colored according to the day after implantation;
**c.** t-SNE projection of the learned latents across behavioral tasks, unobserved during training, showing a modeling of lower limb, upper limb, and speech brain concepts;
**d.** performance in decoding hip movement intentions (33.3% chance) on a moment-by-moment basis with data points of 200 ms in length. A Random Forest (RF) decoder trained on the latent features (Latent RF) gains 12 points compared to when trained on the input wavelet features (InputRF), with only 4% difference to a transformer decoder (NeuralT), suggesting that meaningful feature extraction is key;
**e.** performance in 6-class arm direction decoding (16.7% chance) with limited labels (10 per class) of a randomly initialized transformer (RandomT) and a SSL-pre-trained transformer (NeuraIT).

**Fig. 4** schematically shows learning of the natural language of the brain cortex, in particular the motor cortex, in the system 100 of the invention.

Here, reconstruction is carried out on three different brain activity Samples (Samples 1-3).

In particular, **Fig. 4** illustrates network's reconstruction of the three brain activity Samples, including spatio-spectro-temporal information, from their randomly masked sampling.

**Fig. 5** schematically shows learning of meaningful natural representations in the system 100 of the invention.

By projecting the latent features in 3D (using t-SNE, which is a dimensionality reduction method), reveals interesting structures (in the example of **Fig. 5****,** different electrodes configurations), that are less visible when input tensor are projected in 3D.

This suggests that the latent features are potentially more efficient representations compared to the input tensors.

As mentioned, latent embeddings can be seen as a compressed version of the input that is more informative and provides higher performance in downstream tasks.

The latent features can be used in the downstream tasks, such as motor intention classification, compensation of temporal drift, or patient-to-patient generalization.

**Fig. 6** shows and example referring to a movement of the lower limbs of a patient P, in particular walking.

Here, the example demonstrates how the system 100 of the invention allows achieving highly-decodable and efficient feature extraction. In particular, **Fig. 6** shows the following:
**a.** t-SNE projection of the original input features and the latent features showing increased segregation in the latent embeddings of the left and right hip movement intentions;
**b.** On the left, performance in hip movement intention decoding (33.3% chance) with increasing number of training cues. A classical decoder (Random Forest), trained on the latent features, shows increased performance compared to when trained on the input features, especially with low number of training labels. On the right, decoding probability peaks after movement onsets, showing that the decoder trained on the latent features, provides more confident predictions compared to the input model, even when the number of given cues is low.

A further example illustrating how the system 100 allows achieving highly-decodable and efficient feature extraction is shown in **Fig. 7****.**

Here, the example refers to robot-driven movements of the upper limbs of a patient P.

In particular, **Fig. 7** shows the following:
**d.** experimental setup of the task involving a robotic arm assisting the patient's movements;
**e.** Performance of a Random Forest (RF) decoder trained on the latent features or the input features as a function of the percentage of the available training cues;
**f.** t-SNE projection of the original input features and the latent features showing increased segregation in the latent embeddings corresponding to different upper limb joints involved in the movement;
**g.** Decoding probabilities across time in three different states (shoulder, elbow, hand);
**h.** Confusion matrices in 8 states upper limb classification of a RF trained on either the latent or the input features, showing a gain of 13 points in accuracy when using the latent features.

**Fig. 8** shows an example of models distribution shifts across time, the context of the present invention.

Here, projection of pre-training epochs across time is shown, with each data point colored according to the day after implantation.

In particular, **Fig. 8** shows the following:
**a.** t-SNE projection of the learned latents with pre-training epochs across time, with each data point colored according to the day after implantation;
**b.** distance in latent space, of each day's centroid, relative to the centroid of day 167 after implantation;
**c.** PCA projection of the learned latents before and after unsupervised alignment of second order statistics, with each data point colored according to the day after implantation.

An example of raw latent embeddings and aligned latent embeddings across time is shown in detail in **Fig. 9****.**

Here, the example refers to a right hip movement, a left hip movement, and a rest condition.

In particular, **Fig, 9** shows the t-SNE projections of the learned latents before and after supervised alignment, using a contrastive objective based on the behavioral labels of left hip, right hip, and resting state.

The left part of the figure illustrates the non-aligned latent features, showing a change in the neural dynamics across days for the different behavioral conditions.

The right part of the figure illustrates the latent space obtained after contrastive alignment trained on day 1 to day 10 of rehabilitation (projected from day 1 to day 32 of rehabilitation), suggesting the possibility of obtaining a latent space that is robust and invariant to shifts in neural distributions from session to session,

As mentioned, the system 100 of the invention can be used in online, real-time clinical settings.

An online, real-time clinical settings experiment was conducted with a human patient to demonstrate this principle.

The results of this experiment are shown in **Fig. 10****.**

In particular, the experiment revealed the ability of the system 100 to learn a complex 6-states upper limb task based on latent features, in online, real-time clinical settings.

The present invention further provides a method for planning neuromodulation and/or neurostimulation, in particular with the system 100 described above.

In particular, the method comprises:
- providing at least one input module 10 for brain signals S, especially raw brain signals S, preferably wherein said brain signals S at least partially include cortical brain signals S, more preferably unlabeled cortical brain signals S,
- converting the raw brain signals S into input tensors, preferably 3D input tensors, through a pre-processing module 20, said input tensors including at least one of temporal and/or spatial and/or spectral information,
- providing at least one conversion module 12, said conversion module 12 comprising an encoder and decoder network architecture 14,
- training the encoder and decoder network architecture 14 by using partially masked samples of the input tensors to reconstruct a given representation of the input tensors.

A masking ratio is applied with positional embedding, which provides simultaneous learning of local and global features present in the brain data, thereby transforming the input tensors into a latent space.

In the present embodiment, said brain signals S at least partially include cortical brain signals S, more preferably unlabeled cortical brain signals S.

In the present embodiment, said input tensors are 3D input tensors.

Preferably, the dimensions of the 3D input tensors include at least one of spatial, temporal and/or spectral information.

In the present embodiment, the method further comprises performing a self-supervised learning (SSL).

This step is implemented through the encoder and decoder network architecture 14 described above.

In the present embodiment, the method further comprises:
- learning patterns of unlabeled neural activity, thereby at least partially uncovering the underlying dynamical structure of the brain B, and
- establishing a generic latent space that correlates with behavior in varying contexts.

These steps are as well implemented through the encoder and decoder network architecture 14 described above.

In the present embodiment, the step of converting the brain signals S into input tensors is conveniently implemented by using a wavelet transformation.

In the present embodiment, the method further comprises transforming the brain signals S into a latent space.

This step is implemented thorough the encoder module 16 described above.

The method further comprises:
- mapping the latent space to the original feature space, and
- computing the reconstruction loss.

These steps are implemented thorough the decoder module 18 described above.

The present invention further provides a computer-readable non-volatile storage medium comprising computer-readable instructions that, when executed by a processing means, causes said processing means to implement the method steps described above.

### References

- 100: System

- 10: Input module
- 12: Conversion module
- 14: Encoder / decoder network architecture
- 16: Encoder module
- 18: Decoder module
- 20: Pre-processing module

- B: Brain
- D: Brain recording devices (recording electrodes)
- S: Brain signals
- P: Patient

### Bibliography

**[1]** J. Devlin, M.-W. Chang, K. Lee, and K. Toutanova, "BERT: Pre-training of DeepBi-directional Transformers for Language Understanding." arXiv, May 24, 2019. doi: 10.48550/arXiv.1810.04805.
**[2]** T. B. Brown et al., "Language Models are Few-Shot Learners." arXiv, Jul. 22, 2020. doi:10.48550/arXiv.2005.14165.
**[3]** OpenAI, "GPT-4 Technical Report." arXiv, Mar. 27, 2023. doi: 10.48550/arXiv.2303.08774.
**[4]** M. Popel et al., "Transforming machine translation: a deep learning system reaches news translation quality comparable to human professionals," Nat. Commun., vol. 11, no. 1, Art. no. 1, Sep. 2020, doi: 10.1038/s41467-020-18073-9.
**[5]** M. Oquab et al., "DINOv2: Learning Robust Visual Features without Supervision." arXiv, Apr. 14, 2023. doi: 10.48550/arXiv.2304.07193.
**[6]** K. He, X. Chen, S. Xie, Y. Li, P. Dollár, and R. Girshick, "Masked Autoencoders Are Scalable Vision Learners." arXiv, Dec. 19, 2021. doi: 10.48550/arXiv.2111.06377.
**[7]** Z. Xie et al., "SimMIM: A Simple Framework for Masked Image Modeling." arXiv, Apr. 17, 2022. doi: 10.48550/arXiv.2111.09886.
**[8]** P.-Y. Huang et al., "Masked Autoencoders that Listen." arXiv, Jan. 12, 2023. doi:10.48550/arXiv.2207.06405.
**[9]** H. Banville, I. Albuquerque, A. Hyvärinen, G. Moffat, D.-A. Engemann, and A. Gramfort, "Self-Supervised Representation Learning from Electroencephalography Signals," in 2019 IEEE 29th International Workshop on Machine Learning for Signal Processing (MLSP), Oct. 2019, pp. 1-6. doi: 10.1109/MLSP.2019.8918693.
**[10]** D. Kostas, S. Aroca-Ouellette, and F. Rudzicz, "BENDR: using transformers and a contrastive self-supervised learning task to learn from massive amounts of EEG data." arXiv, Jan. 28, 2021. doi: 10.48550/arXiv.2101.12037.
**[11]** M. N. Mohsenvand, M. R. Izadi, and P. Maes, "Contrastive Representation Learning for Electroencephalogram Classification," in Proceedings of the Machine Learning for Health NeurIPS Workshop, PMLR, Nov. 2020, pp. 238-253. Accessed: Jul. 01, 2023. [Online]. Available: https://proceedings.mlr.press/v136/mohsenvand20a.html
**[12]** H.-Y. S. Chien, H. Goh, C. M. Sandino, and J. Y. Cheng, "MAEEG: Masked Auto-encoder for EEG Representation Learning." arXiv, Oct. 27, 2022. doi: 10.48550/arXiv.2211.02625.
**[13]** C. Pandarinath et al., "Inferring single-trial neural population dynamics using sequential auto-encoders," Nat. Methods, vol. 15, no. 10, Art. no. 10, Oct. 2018, doi:10.1038/s41592-018-0109-9.
**[14]** M. R. Keshtkaran et al., "A large-scale neural network training framework for generalized estimation of single-trial population dynamics," Nat. Methods, vol. 19, no. 12, Art. no. 12, Dec. 2022, doi: 10.1038/s41592-022-01675-0.
**[15]** S. Schneider, J. H. Lee, and M. W. Mathis, "Learnable latent embeddings for joint behavioural and neural analysis," Nature, vol. 617, no. 7960, Art. no. 7960, May 2023, doi: 10.1038/s41586-023-06031-6.
**[16]** C. Wang et al., "BrainBERT: Self-supervised representation learning for intracranial recordings." arXiv, Feb. 28, 2023. doi: 10.48550/arXiv.2302.14367.

## Claims

1. A system (100) for planning and/or providing control to neuromodulation and/or neurostimulation and/or an actuator such as a brain computer interface (BCI) and/or providing a neural interface system, especially a brain-spinal-cord-interface system, the system (100) comprising:
- at least one input module (10) for brain signals (S), especially raw brain signals (S),
- at least one pre-processing module (20) for converting the raw brain signals (S) into input tensors, wherein the input tensors include at least one of temporal and/or spatial and/or spectral information, and
- at least one conversion module (12) comprising an encoder and decoder network architecture (14),
- wherein the encoder and decoder network architecture (14) is configured to be trained by using partially masked samples of the input tensors to reconstruct electrical activity of a given representation of the input tensors, wherein a masking ratio is applied with positional embedding, which provides simultaneous learning of local and global features present in the brain data, thereby transforming the input tensors into a latent space.

2. The system (100) according to claim 1, **characterized in that** the encoder and decoder network architecture (14) is configured to perform self-supervised learning (SSL).

3. The system (100) according to claim 1 or claim 2, **characterized in that** the encoder and decoder network architecture (14) is configured to learn patterns of unlabeled neural activity, thereby at least partially uncovering the underlying dynamical structure of the brain (B) and to establish a generic latent space that correlates with behavior in varying contexts.

4. The system (100) according to any one of the preceding claims, **characterized in that** the brain signals (S) comprise at least partially cortical brain signals (S), especially unlabeled cortical brain signals (S).

5. The system (100) according to any one of the preceding claims, **characterized in that** the input tensors are 3D input tensors, preferably wherein the dimensions include at least one of spatial, temporal and/or spectral information.

6. The system (100) according to any one of the preceding claims, **characterized in that** the pre-processing module (20) is configured to use a wavelet transformation to convert the brain signals (S) into input tensors.

7. The system (100) according to any one of the preceding claims, **characterized in that** the encoder and decoder network architecture (14) comprises an encoder module (16), which is configured to compress the brain signals (S) into a latent space.

8. The system (100) according to claim 7, **characterized in that** the encoder and decoder network architecture (14) comprises a decoder module (18), which is configured to map the latent space to the original feature space and to compute the reconstruction loss.

9. The system (100) according to any one of the preceding claims, **characterized in that** the masking ratio is a high masking ratio with positional embedding.

10. A method for planning neuromodulation and/or neurostimulation through the system (100) according to any one of the preceding claims, the method comprising:
- providing at least one input module (10) for brain signals (S), especially raw brain signals (S), preferably wherein said brain signals (S) at least partially include cortical brain signals (S), more preferably unlabeled cortical brain signals (S),
- converting the raw brain signals (S) into input tensors, preferably 3D input tensors, through a pre-processing module (20), said input tensors including at least one of temporal and/or spatial and/or spectral information,
- providing at least one conversion module (12), said conversion module (12) comprising an encoder and decoder network architecture (14),
- training the encoder and decoder network architecture (14) by using partially masked samples of the input tensors to reconstruct a given representation of the input tensors,
- wherein a masking ratio is applied with positional embedding, which provides simultaneous learning of local and global features present in the brain data, thereby transforming the input tensors into a latent space.

11. The method according to claim 10, **characterized in that** the method further comprises performing self-supervised learning (SSL).

12. The method according to claim 10 or claim 11, **characterized in that** the method further comprises:
- learning patterns of unlabeled neural activity, thereby at least partially uncovering the underlying dynamical structure of the brain (B), and
- establishing a generic latent space that correlates with behavior in varying contexts.

13. The method according to any one of claims 10 to 12, **characterized in that** the step of converting the brain signals (S) into input tensors is implemented by using a wavelet transformation.

14. The method according to any one of claims 10 to 13, **characterized in that** the method further comprises:
- compressing the brain signals (S) into a latent space,
preferably wherein the method further comprises:
- mapping the latent space to the original feature space, and
- computing the reconstruction loss.

15. A computer-readable non-volatile storage medium comprising computer-readable instructions that, when executed by a processing means, causes said processing means to implement the method steps defined in claims 10 to 14.
